Europäisches Patentamt

European Patent Office

Office européen des brevets

(19)

(11) Veröffentlichungsnummer: **0 370 997**
**A2**

## (12) EUROPÄISCHE PATENTANMELDUNG

(21) Anmeldenummer: **89890299.4**

(22) Anmeldetag: **16.11.89**

(51) Int. Cl.⁵: **A61M 39/00, A61M 39/04**

(30) Priorität: **22.11.88 AT 2864/88**

(43) Veröffentlichungstag der Anmeldung:
**30.05.90 Patentblatt 90/22**

(84) Benannte Vertragsstaaten:
**BE CH DE ES GB GR IT LI NL SE**

(71) Anmelder: **Hirnich, Paul**
**Zwölfergasse 4**
**A-1150 Wien(AT)**

(72) Erfinder: **Hirnich, Paul**
**Zwölfergasse 4**
**A-1150 Wien(AT)**

(74) Vertreter: **Kliment, Peter, Dipl.-Ing. Mag.-jur.**
**Singerstrasse 8/3/8**
**A-1010 Wien(AT)**

(54) **Anschlussstück für eine Infusionseinrichtung od.dgl.**

(57) Anschlußstück für eine Infusionseinrichtung od. dgl. mit einer, in einem vorzugsweise aus einem durchsichtigen oder durchscheinenden Kunststoff hergestellten Gehäuse (1) gehaltenen Kanüle, welches Gehäuse (1) einen mit radial abstehenden Anschlüssen (5, 6, 7) versehenen Hohlraum (3) aufweist, in dem eine die Bohrungen dieser Anschlüsse (5, 6, 7) abschließende hohle Kappe (4) aus einem elastischen Material angeordnet ist und bei dem Anschlußnippel (15) von Schläuchen in die Bohrungen der Anschlüsse (5, 6, 7) des Gehäuses (1) einsetzbar sind, wobei die Kappe (4) den Anschluß (5, 6, 7) des Gehäuses, in dem ein Anschlußnippel (15) eines Schlauches od. dgl. eingesetzt ist, freigibt. Um einen stets gleichbleibenden Durchtrittsquerschnitt für die Infusionsflüssigkeit, sicherzustellen ist vorgesehen, daß in jedem radial abstehenden Anschluß (5, 6, 7) des Gehäuses (1) ein mit einem Kanal versehenes Gleitstück (8) gehalten ist, das mit seinem einen Ende auf der Mantelfläche der Kappe (4) aufsteht und an dessen anderer Stirnseite das Anschlußnippel (15) zur Anlage bringbar ist, wobei die Länge des Gleitstückes (8) größer als der Abstand zwischen der Mantelfläche der Kappe (4) und der freien Stirnfläche des Anschlußnippels (15) in dessen voll in die Bohrung eines Anschlusses (5, 6, 7) eingesetzter Lage ist.

Fig. 1

Die Erfindung bezieht sich auf ein Anschlußstück für eine Infusionseinrichtung od. dgl. mit einer, in einem vorzugsweise aus einem durchsichtigen oder durchscheinenden Kunststoff hergestellten Gehäuse gehaltenen Kanüle, welches Gehäuse einen mit radial abstehenden Anschlüssen versehenen Hohlraum aufweist, in dem eine die Bohrungen dieser Anschlüsse abschließende hohle Kappe aus einem elastischen Material angeordnet ist und bei dem Anschlußnippel von Schläuchen in die Bohrungen der Anschlüsse des Gehäuses einsetzbar sind, wobei die Kappe den Anschluß des Gehäuses, in dem ein Anschlußnippel eines Schlauches od. dgl. eingesetzt ist, freigibt.

Ein solches Anschlußstück wurde z.B. durch die DE-OS 31 42 524 bekannt. Bei dieser bekannten Lösung ist die aus einem elastisch verformbaren Material hergestellte Kappe lediglich an einer Stelle mit der Innenwand des Gehäuses verbunden, wobei die Wand dieser Kappe bei in die Anschlüsse des Gehäuses eingesetzten Anschlußnippeln der Schläuche von deren Dichtsitz weggedrückt wird.

Dabei ergibt sich allerdings der Nachteil, daß die dadurch freigelegte Durchtrittsöffnung, abhängig von dem jeweiligen Anschlußnippel, unterschiedlich groß werden kann. Dies ist insbesondere dann der Fall, wenn in zwei Anschlüsse des Gehäuses Anschlußstücke von Schläuchen eingesetzt werden.

Außerdem kann es durch das direkte Ansetzen der Anschlußnippel, die in der Regel einen zentralen Kanal aufweisen, zu einem weitgehenden Verschließen desselben durch die Wand der Kappe kommen, wodurch es zu Schwierigkeiten bei der Infusion kommen kann.

Ziel der Erfindung ist es diese Nachteile zu vermeiden und ein Anschlußstück der eingangs erwähnten Art vorzuschlagen, bei dem auf einfache Weise der Zutritt der Infusionsflüssigkeit in das Innere des Gehäuses in praktisch stets gleicher Weise sichergestellt ist.

Erfindungsgemäß wird dies dadurch erreicht, daß in jedem radial abstehenden Anschluß des Gehäuses ein mit einem Kanal versehenes Gleitstück gehalten ist, das mit einer Stirnseite auf der Mantelfläche der Kappe aufsteht und an dessen anderer Stirnseite das Anschlußnippel zur Anlage bringbar ist, wobei die Länge des Gleitstückes größer als der Abstand zwischen der Mantelfläche der Kappe und der freien Stirnfläche des Anschlußnippels in dessen voll in die Bohrung eines Anschlusses eingesetzter Lage ist.

Dadurch ist ein sicheres Öffnen der Verbindung zwischen dem Inneren des Gehäuses und dem Anschluß des Gehäuses gewährleistet.

Um den Abfluß der Infusionsflüssigkeit zu erleichtren kann weiters vorgesehen sein, daß die von der Kappe abgekehrte Stirnseite des Gleitstükkes zumindest in dem der Kanüle näheren Bereich des Gleitstückes schräg gegen die Längsachse des Gleitstückes geneigt verläuft, wobei diese Neigung in Richtung der der Kappe zugekehrten Stirnseite abfällt.

Damit ist ein Verschließen des Kanals des Anschlußnippels ausgeschlossen.

Bei einer bevorzugten Ausführungsform der Erfindung ist vorgesehen, daß das Gleitstück an seiner von der Kappe abgekehrten Stirnseite einen seitlich vorstehenden Ansatz aufweist, der im Querschnitt vorzugsweise sichelförmig ausgebildet ist und in einer im wesentlichen gegengleichen. Nut des Anschlusses geführt ist.

Dadurch ergibt sich eine sichere Führung und es wird gleichzeitig sichergestellt, daß das Gleitstück stets an der selben Stelle auf die ähnlich einem Klappenventil wirkende Kappe auftrifft, wenn ein Anschlußnippel in den Anschluß des Gehäuses eingesetzt wird.

Weiters ist es aus Gründen einer einfacheren Herstellung günstig, wenn der Kanal des Gleitstükkes durch eine in axialer Richtung durchgehende Nut gebildet ist, die vorzugsweise diametral zum seitlich vorstehenden Vorsprung angeordnet ist.

Weiters kann vorgesehen sein, daß die der Kappe zugekehrte Stirnseite des Gleitstückes einen von 90° abweichenden Winkel mit seiner Längsachse einschließt, wobei die Nut des Gleitstückes vorzugsweise in dem der Kanüle näheren Bereich des Gleitstückes angeordnet ist.

Auf diese Weise wird eine entsprechende Freistellung im Bereich des der Kappe näheren Endes des Gleitstückes gewahrleistet.

Um ein Herausrutschen der Gleitstücke, die mit einem entsprechenden Spiel in den Bohrungen der Anschlüsse des Gehäuses gehalten sind, zu vermeiden kann weiters vorgesehen sein, daß in der den seitlichen Vorsprung des Gleitstückes aufnehmenden Nut des Anschlusses des Gehäuses zwei in axialer Richtung voneinander distanzierte Anschläge vorgesehen sind, zwischen denen der seitliche Vorsprung des Gleitstückes verschiebbar ist und von denen der dem freien Ende des Anschlusses nähere Anschlag vom Gleitstück elastisch überdrückbar ist.

Die Erfindung wird nun anhand der Zeichnung näher erläutert. Dabei zeigen:

Fig. 1 einen Schnitt entlang der Linie I-I in Fig. 2 durch ein erfindungsgemäßes Anschlußstück,

Fig. 2 einen Schnitt entlang der Linie II-II in der Fig. 1,

Fig. 3 einen Schnitt gemäß der Fig. 1 bei eingedrücktem Gleitstück.

Fig. 4 einen Schnitt durch das Gleitstück, und

Fig. 5 eine Draufsicht auf das Gleitstück.

Das Gehäuse 1 weist eine Aufnahme 2 für eine nicht dargestellte Kanüle auf, die sich bis in den Hohlraum 3 erstreckt, der durch eine Kappe 4 aus einem elastisch verformbaren Material, wie z.B. Silikongummi, abgeschlossen ist.

Diese Kappe 4 ist im Bereich ihrer vollen Stirnwand 17 mit der Innenwand des Gehäuses 1 verbunden, z.B. verklebt und deckt mit ihrer zylindrischen Wand auch die Einmündungen der Bohrungen der Anschlüsse 5, 6 und 7, ähnlich einem Klappenventil ab.

In jedem dieser Anschlüsse 5 bis 7, die radial vom Gehäuse 1 abstehen, ist ein, im wesentlichen zylindrisches Gleitstück 8 axial verschiebbar in dessen Bohrung gehalten und steht mit seiner einen Stirnfläche auf der zylindrischen Wand der Kappe 4 auf.

Die der Kappe 4 zugekehrten Stirnseiten der Gleitstücke 8 sind abgeschrägt, um einen entsprechenden Freiraum für den Durchtritt der Infusionsflüssigkeit zwischen der Kappe 4 und der Innenwand des Hohlraumes 3 des Gehäuses 1 zu schaffen.

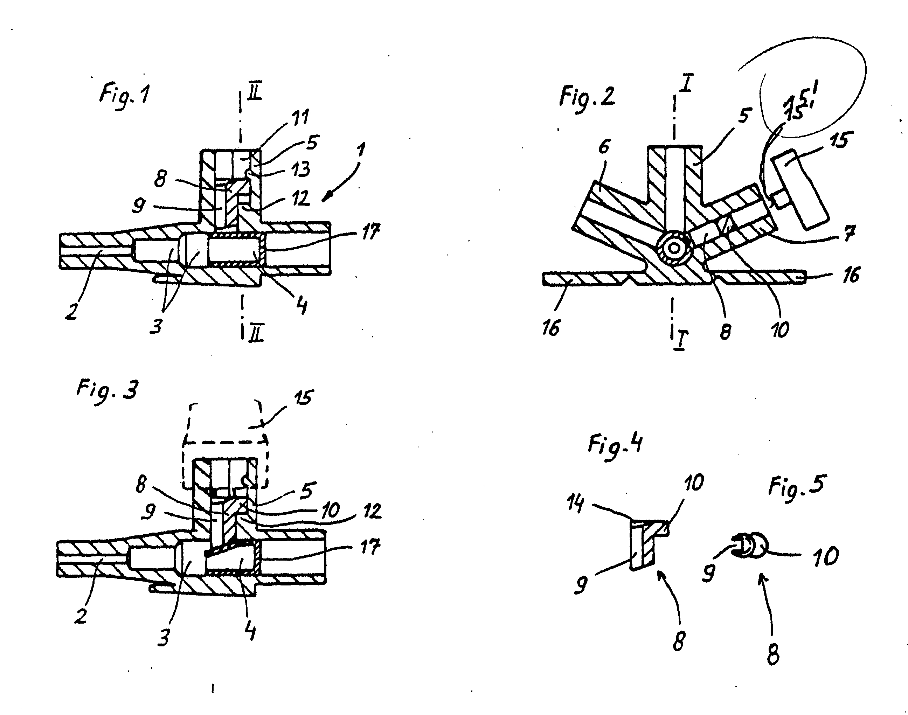

Die Gleitstücke 8 weisen je eine Nut 9 auf, die als Kanal für die Infusionsflüssigkeit dient und sich über die gesamte Länge des Gleitstückes erstreckt. Weiters weisen die Gleitstücke 8 an deren von der Kappe 4 abgekehrten Enden je einen seitlich vorstehenden Ansatz 10 auf, der wie insbesondere aus der Fig. 5 zu ersehen ist, im wesentlichen sichelförmig ausgebildet ist, bzw. mit dem Querschnitt des übrigen Gleitstückes eine sog. "Double Bubble Form" ergibt.

Dieser seitliche Ansatz 10 gleitet in einer in axialer Richtung verlaufenden Nut 11 der Bohrungen der Anschlüsse 5, 6 und 7 des Gehäuses, die allerdings vor der Einmündung der entsprechenden Bohrung des Anschlusses in den Hohlraum 3 des Gehäuses 1 endet und die dadurch gegebene Schulter 12 einen Anschlag für den Ansatz 10 des Gleitstückes 8 bildet, der den Bewegungsweg des Gleitstückes begrenzt.

Weiters ragt ein zweiter Anschlag 13 in Form einer kleinen Noppe in die Nut 11 des Anschlusses hinein, der ein Herausrutschen des Gleitstückes 8 sicher verhindert. Dieser Anschlag 13 kann jedoch aufgrund der Elastizität des für die Herstellung des Gehäuses 1 verwendeten Materials, wie z.B. Kunststoff, beim Einsetzen des Gleitstückes in den Anschluß überdrückt werden.

Beim dargestellten Ausführungsbeispiel weist die von der Kappe 4 abgekehrte Stirnfläche des Gleitstückes 8 abschnittweise eine Neigung auf, die durch die schräg verlaufende Rinne 14 gebildet ist. Dadurch steht beim Einsetzen eines Anschlußnippels 15 in den Anschluß 5, 6 oder 7, dieses mit derStirnfläche 15′ seines zentral angeordneten Kanals auf den Rändern der Rinne 14 auf und die Flüssigkeit kann leicht aus dem Anschlußnippel, das zumeist an einem Schlauch montiert ist, austreten. Dabei verläuft der Boden der Rinne 14 gegen die Nut 9 zu nach unten bzw. in Richtung zur zweiten Stirnseite des Gleitstückes 8 hin geneigt.

Beim Einsetzen eines Anschlußnippels 15 in einen Anschluß 5, 6 oder 7, kommt dieser an der einen Stirnseite des Gleitstückes 8 zur Anlage und drückt diese gegen die Wand der Kappe 4, sodaß sich diese von der Innenwand des Hohlraumes 3 abhebt und eine Passage für die Infusionsflüssigkeit freigibt. Dabei kommt der seitliche Ansatz 10 des Gleitstückes 8 an der Schulter 12 zur Anlage, wie dies aus der Fig. 3 zu ersehen ist, in der das Anschlußnippel 15 strichliert angedeutet ist. Der Abstand zwischen der der Kappe 4 zugekehrten Fläche des Ansatzes 10 von der auf der Kappe 4 aufstehenden Kante der schräg geneigten Stirnfläche des Gleitstückes 8 ist größer, als der Abstand der Schulter 12 zur zylindrischen Wand der Kappe 4.

Das Anschlußnippel 15 kann mittels eines nicht dargestellten Gewindes oder eines Bajonettverschlusses mit dem radial abstehenden Anschluß 5, 6 oder 7 verbunden werden.

Zur leichteren Befestigung an einem Patienten sind an das Gehäuse 1 Flügel 16 angespritzt.

Weiters kann die Stirnwand 17 der Kappe 4 leicht durchstochen werden, sodaß mittels einer Injektionsnadel z.B. ein Medikament in den Hohlraum 3, zusätzlich zu Infusionen eingebracht werden kann. Desgleichen kann mit einer entsprechend langen Injektionsnadel, die sich auch durch die zweckmäßigerweise biegsame und z.B. aus Tetrafluorkohlenstoffverbindungen hergestellte Kanüle durchsetzt, das Anschlußstück in eine Vene oder Arterie des Patienten eingesetzt werden, wonach die Nadel wieder herausgezogen werden kann. Das dabei entstehende Loch in der durchgehenden Stirnwand 17 der Kappe 4 schließt sich aufgrund deren Elastizität soweit, daß es zu keinem Flüssigkeitsaustritt kommt, insbesondere wenn für die Kappe Silikonkautschuk verwendet wird.

## Ansprüche

1. Anschlußstück für eine Infusionseinrichtung od. dgl. mit einer, in einem vorzugsweise aus einem durchsichtigen oder durchscheinenden Kunststoff hergestellten Gehäuse gehaltenen Kanüle, welches Gehäuse einen mit radial abstehenden Anschlüssen versehenen Hohlraum aufweist, in dem eine die Bohrungen dieser Anschlüsse abschließende hohle Kappe aus einem elastischen Material angeordnet ist und bei dem Anschlußnippel von Schläuchen in die Bohrungen der Anschlüsse des Gehäuses einsetzbar sind, wobei die Kappe den Anschluß des Gehäuses, in dem ein Anschlußnippel eines Schlauches od. dgl. eingesetzt ist, freigibt, dadurch gekennzeichnet, daß in jedem radial abstehenden Anschluß (5, 6, 7) des Gehäuses (1) ein mit einem Kanal versehenes Gleitstück (8) gehalten ist, das mit seiner Stirnseite auf der Mantelfläche der Kappe (4) aufsteht und an dessen anderer Stirnseite das Anschlußnippel (15) zur Anlage bringbar ist, wobei die Länge des Gleitstückes (8) größer als der Abstand zwischen der Mantelfläche der Kappe (4) und der freien Stirnfläche (15') des Anschlußnippels (15) in dessen voll in die Bohrung eines Anschlusses (5, 6, 7) eingesetzter Lage ist.

2. Anschlußstück nach Anspruch 1, dadurch gekennzeichnet, daß das Gleitstück (8) an seiner von der Kappe (4) abgekehrte Stirnseite einen seitlich vorstehenden Ansatz (10) aufweist, der im Querschnitt vorzugsweise sichelförmig ausgebildet ist und in einer im wesentlichen gegengleichen Nut (11) der Bohrung des Anschlusses (5, 6, 7) geführt ist.

3. Anschlußstück nach Anspruch 1 oder 2, dadurch gekennzeichnet, daß der Kanal des Gleitstückes (8) durch eine in axialer Richtung durchgehende Nut (9) gebildet ist, die vorzugsweise diametral zum seitlich vorstehenden Vorsprung (10) angeordnet ist.

4. Anschlußstück nach einem der Ansprüche 1 bis 3, dadurch gekennzeichnet, daß die der Kappe (4) zugekehrte Stirnseite des Gleitstückes (8) einen von 90° abweichenden Winkel mit seiner Längsachse einschließt, wobei die Nut (9) des Gleitstückes (8) vorzugsweise in dem der Kanüle näheren Bereich des Gleitstückes (8) angeordnet ist.

5. Anschlußstück nach einem der Ansprüche 1 bis 4, dadurch gekennzeichnet, daß in der den seitlichen Vorsprung (10) des Gleitstückes (8) aufnehmenden Nut (11) der Bohrung des Anschlusses (5, 6, 7) des Gehäuses (1) zwei in axialer Richtung voneinander distanzierte Anschläge (12, 13) vorgesehen sind, zwischen denen der seitliche Vorsprung (10) des Gleitstükkes (8) verschiebbar ist und von denen der dem freien Ende des Anschlusses (5, 6, 7) nähere Anschlag (13) vom Gleitstück (8) elastisch überdrückbar ist.

6. Anschlußstück nach einem der Ansprüche 1 bis 5, dadurch gekennzeichnet, daß die von der Kappe (4) abgekehrte Stirnseite des Gleitstückes (8) zumindest in dem der Kanüle näheren Bereich des Gleitstückes (8) schräg gegen die Längsachse des Gleitstückes (8) geneigt verläuft, wobei diese Neigung in Richtung der der Kappe (4) zugekehrten Stirnseite des Gleitstückes (8) abfällt.

Fig.1

Fig.2

Fig.3

Fig.4

Fig.5